(19) Europäisches Patentamt
European Patent Office
Office européen des brevets

(11) Veröffentlichungsnummer: **0 065 294**
**A1**

(12) # EUROPÄISCHE PATENTANMELDUNG

(21) Anmeldenummer: **82104243.9**

(22) Anmeldetag: **14.05.82**

(51) Int. Cl.³: **C 07 C 121/46**

(30) Priorität: **19.05.81 DE 3119819**

(43) Veröffentlichungstag der Anmeldung: **24.11.82**
**Patentblatt 82/47**

(84) Benannte Vertragsstaaten: **BE CH DE FR GB IT LI NL**

(71) Anmelder: **HOECHST AKTIENGESELLSCHAFT,**
**Postfach 80 03 20, D-6230 Frankfurt/Main 80 (DE)**

(72) Erfinder: **Röhrscheid, Freimund, Dr., Amselweg 24,**
**D-6233 Kelkheim (Taunus) (DE)**
Erfinder: **Riemenschneider, Wilhelm, Dr.,**
***Loreleistrasse 45, D-6230 Frankfurt am Main 80 (DE)***

(54) **Verfahren zur Herstellung von Formylcyannorbornan.**

(57) Formylcyannorbornan wird hergestellt durch Hydroformylierung von Cyannorbornen mit einem $H_2$/CO-Gasgemisch eines (Mol)-Verhältnisses von $H_2$:CO von grösser als 1, vorzugsweise von etwa 1,5 bis 5, insbesondere von etwa 1,5 bis 3.

Das Verfahren arbeitet mit hoher Selektivität und Ausbeute.

Das Verfahrensprodukt Formylcyannorbornan ist hauptsächlich Zwischenprodukt auf verschiedenen Sachgebieten wie z.B. auf dem Polymerensektor.

EP 0 065 294 A1

## Verfahren zur Herstellung von Formylcyannorbornan

Unter Formylcyannorbornan werden hier die beiden Verbindungen I und II

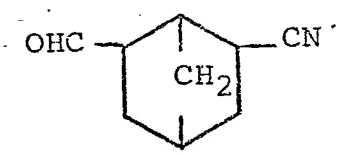

(I)

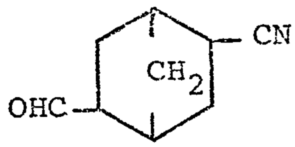

(II)

sowohl einzeln als auch in Mischung miteinander verstanden.

Formylcyannorbornan ist hauptsächlich ein wertvolles Zwischenprodukt auf verschiedenen Sachgebieten wie z.B. auf den Polymeren-Gebiet; es kann z.B. nach dem in der US-PS 3 143 570 beschriebenen Verfahren einer aminierenden Hydrierung (mit $NH_3/H_2$) zu dem entsprechenden Diamin unterworfen und dieses dann mit Di- oder Polycarbonsäuren zu entsprechenden Polyamiden kondensiert werden.

In der erwähnten US-PS ist auch eine Methode zur Herstellung des Formylcyannorbornans angegeben. Diese Methode geht aus von dem - seinerseits etwa durch Diels-Alder-Reaktion aus Cyclopentadien und Acrylnitril erhältlichen - Cyannorbornen III, welches mit einem $H_2$/CO-Gasgemisch ("Synthesegas") in Gegenwart von carbonylbildenden Metallen der VIII. Gruppe des Periodensystems der Elemente, oder von Verbindungen solcher Metalle - insbesondere von fein verteiltem Kobalt oder von Kobaltverbindungen - bei Temperaturen zwischen etwa 90 und etwa 150°C und einem Druck zwischen etwa 1.000 und 4.000 psi (entsprechend etwa 70 bis 280 bar) oder höher in Gegenwart oder in Abwesenheit eines Lösungsmittels hydroformyliert wird. Diese Reaktion verläuft nach folgender Reaktionsgleichung:

0065294

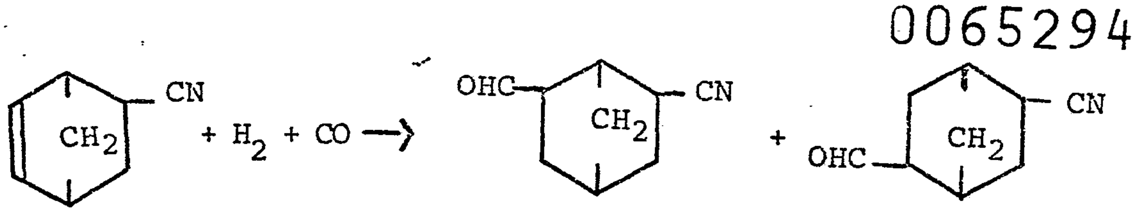

(III) (I) (II)

Nach dem einzigen in der US-PS enthaltenden Beispiel für die Durchführung dieser Reaktion (Example I) werden 68 % d. Th. an Roh-Formylcyannorbornan und - wie sich aus den dortigen Zahlenwerten errechnet - von 57,5 % d. Th. an destilliertem Produkt erhalten. In dem Beispiel ist über das verwendete $H_2$/CO-Verhältnis allerdings nichts ausgesagt. Da bei der Nacharbeitung dieses Beispiels mit dem - an sich nächstliegenden - stöchiometrischen Verhältnis ($H_2$/CO = 1 : 1) jedoch praktisch genau die dort angegebene Ausbeute an Formylcyannorbornan erhalten wurde, dürfte von den Autoren der US-PS wohl auch mit diesem Verhältnis gearbeitet worden sein. Wegen der insbesondere für eine technische Durchführung kaum befriedigenden Ausbeute an Formylcyannorbornan sowie auch einem beträchtlichen Anfall an (unerwünschten) Harzprodukten bestand die Aufgabe, das in der vorerwähnten US-PS beschriebene Verfahren in Richtung auf eine erhöhte Selektivität des Formylcyannorbornans hin zu verbessern, so daß also eine höhere Ausbeute an Formalcyannorbornan und weniger Harzprodukte erhalten werden.

Diese Aufgabe konnte erfindungsgemäß dadurch gelöst werden, daß man die Hydroformylierung des Cyannorbornens mit einem $H_2$/CO-Verhältnis von größer als 1 durchführt.

Erfindungsgegenstand ist somit ein Verfahren zur Herstellung von Formylcyannorbornan durch Hydroformylierung von Cyannorbornen mit einem $H_2$/CO-Gasgemisch in Gegenwart von fein verteilten Metallen der VIII. Gruppe des Periodensystems der Elemente oder von Carbonylen oder carbonylbildenden Verbindungen dieser Metalle als Katalysatoren bei erhöhter Temperatur und erhöhtem Druck

in Gegenwart oder in Abwesenheit eines inerten Lösungsmittels;
das Verfahren ist dadurch gekennzeichnet, daß man als $H_2$/CO-Gasgemisch ein solches mit einem $H_2$/CO-(Mol)-Verhältnis von größer als 1, vorzugsweise von etwa 1,5 bis 5, insbesondere von etwa 1,5 bis 3, verwendet.
Durch diese Abänderung des in der US-PS 3 143 570 beschriebenen Verfahrens wird die Selektivität des Verfahrens nicht unbeträchtlich erhöht; d. h. es wird eine höhere Ausbeute an dem gewünschten Formylcyannorbornan und eine z. T. erheblich geringere Menge an (unerwünschten) Harzprodukten erhalten.
Diese Erhöhung der Selektivität durch ein über das stöchiometrische $H_2$/CO-Verhältnis hinaus erhöhtes $H_2$-Angebot war außerordentlich überraschend, weil man durch ein derartiges überhöhtes $H_2$-Angebot wegen der verschiedenen hydrierbaren Gruppen und Stellen im Ausgangs- und Endprodukt (Ausgangsprodukt Cyannorbornen: olefinische Doppelbindung, Endprodukt Formylcyannorbornan: Aldehydgruppe, sowohl Ausgangs- als auch Endprodukt: CN-Gruppe sowie Möglichkeit der hydrogenolytischen Spaltung des aliphatisch-bicyclischen Ringsystems) eher mit der vermehrten Bildung unerwünschter Neben- und Harzprodukte und damit mit einer geringeren Selektivität (in Richtung auf das Formylcyannorbornan) rechnen mußte.
Die Bedingungen, unter denen das erfindungsgemäße Verfahren ausgeführt wird, sind - bis auf das $H_2$/CO-Verhältnis - zumindest teilweise identisch mit den in der US-PS 3 143 570 für die Hydroformylierung des Cyannorbornens beschriebenen Bedingungen.
So können für das erfindungsgemäße Verfahren im Prinzip die gleichen Katalysatoren wie für die in der US-PS beschriebene Hydroformylierung verwendet werden; d. s. also die fein verteilten Metalle der VIII. Gruppe des Periodensystems der Elemente sowie die Carbonyle und/oder carbonylbildenden Verbindungen dieser Metalle, wobei fein

verteiltes Kobalt, Kobaltcarbonyl und/oder Kobaltverbindungen bevorzugt sind.
Besonders bevorzugte Katalysatoren sind jedoch fein verteiltes Rhodium sowie Rhodiumcarbonyl und/oder Rhodiumcarbonyl bildende Verbindungen wie z. B. Rhodiumacetat oder Rhodiumoctanoat; die Rhodiumkatalysatoren sind in der US-PS nicht namentlich genannt.
Die Katalysator-Konzentration liegt normalerweise zwischen etwa 0,1 und 10 Gew.-%, vorzugsweise zwischen etwa 1 und 5 Gew.-%, bezogen auf das Ausgangs-Cyannorbornen.
Die Reaktionstemperatur ist im Prinzip nicht kritisch und wird üblicherweise zwischen etwa 140 und 170°C gehalten.

Der Arbeitsdruck liegt vorteilhaft zwischen etwa 70 und 600 bar, wobei der Druck bei einem $H_2$/CO-Verhältnis in der Nähe von 1 : 1 mehr im unteren Teil, bei einem $H_2$/CO-Verhältnis in der Nähe von 5 : 1 mehr im oberen Teil des besagten Druckbereichs liegt.
Als inerte Lösungsmittel können im Prinzip alle das Ausgangs- und Endprodukt sowie die Carbonyle der Metalle der VIII. Gruppe des Periodensystems lösenden und bei den Reaktionsbedingungen selbst nicht reagierenden Lösungsmittel verwendet werden. Bevorzugt wird jedoch in Abwesenheit von Lösungsmitteln gearbeitet.
Für den optimalen Ablauf des erfindungsgemäßen Verfahrens ist die intensive Durchmischung der Flüssigphase wichtig. Die Durchmischung kann sowohl durch mechanische als auch durch Gasrührung erfolgen. Besonders vorteilhaft für den technischen Betrieb ist der Einsatz einer an sich bekannten Gasumlaufpumpe.
Wegen der gegenüber der bekannten Verfahrensweise erhöhten Selektivität und Ausbeute (bis etwa 85 % d. Th.) an Formylcyannorbornan sowie der verminderten Mengen an (unerwünschten) Neben- und Harzprodukten stellt die Erfindung einen nicht unerheblichen Fortschritt dar, welcher insbesondere bei der Durchführung des Verfahrens in technischem Maßstab zum Tragen kommt.

Die Erfindung wird nun durch die nachfolgenden Beispiele näher erläutert. Den Erfindungsbeispielen ist ein Vergleichsbeispiel vorangestellt; dieses Vergleichsbeispiel stellt die Nacharbeitung von Beispiel I der US-PS 3 143 570 mit einem $H_2$/CO-Verhältnis von 1 : 1 dar - aus verfahrenstechnischen Gründen lediglich mit der unwesentlichen Abweichung, daß nicht (wie in dem Beispiel der US-PS) zuerst nur Wasserstoff und dann das $H_2$/CO-Gasgemisch in 2 Stufen, sondern das $H_2$/CO-Gasgemisch gleich bei der Reaktionstemperatur und nach Einstellung des Reaktionsdrucks in einer Stufe eingeleitet wurde.
Das erste Erfindungsbeispiel wurde dann unter den gleichen Bedingungen wie das Vergleichsbeispiel - nur mit dem (erfindungsgemäßen) $H_2$/CO-Verhältnis von 3 : 1 durchgeführt.

Vergleichsbeispiel

In einen 5 l-Autoklaven, der mit einem Rührer, Rückflußkühler und Druckhalteventil ausgerüstet war, wurde eine Lösung von 476 g (= 4 Mol) Cyannorbornen und 30 g Dikobaltoctacarbonyl in 2000 ml trockenem Benzol gefüllt. Das Druckhalteventil wurde auf 210 bar (= etwa 3.000 psi) eingestellt und es wurde eine Mischung von $H_2$ und CO im Verhältnis 1 : 1 aufgedrückt. Die Reaktion wurde bei 140°C durchgeführt. Während der Reaktion wurde laufend frisches Gas der vorstehenden Zusammensetzung zugeführt, bis die Reaktion zum Stillstand kam. Die unverzügliche Destillation des Reaktionsgemischs ergab 400 g = 67,1 % d. Th. Formylcyannorbornan - Rohprodukt (Ausbeuteangabe in Beispiel 1 der US-PS 3 143 570: 68 % Rohprodukt).

Erfindungsbeispiele

Beispiel 1

Das vorstehende Vergleichsbeispiel wurde wiederholt - nur mit dem Unterschied, daß das $H_2$/CO-Verhältnis der

eingeleiteten Gasmischung jetzt 3 : 1 betrug. Bei 210 bar und 140°C wurde laufend frisches Gas dieser Zusammensetzung zugeführt, während über das Druckhalteventil etwa 800 l Gas/Stunde herausgefahren wurden.

Die Reaktionsmischung wurde sofort über einen Dünnschichtverdampfer destilliert (1 x unter Normaldruck, dann im Vakuum), um das Benzol und das Harz abzutrennen. Das Rohprodukt (517 g) wurde gaschromatographisch analysiert; es enthielt 458 g = 76,8 % d. Th. an Formylcyannorbornan.

Das Beispiel zeigt, daß für die erhöhte Selektivität und Ausbeute tatsächlich allein das überstöchiometrische $H_2$/CO-Verhältnis verantwortlich ist.

Beispiele 2 bis 4

In einem 5 l-Autoklaven, der mit einem Hub-Schub-Rührer, Rückflußkühler und Druckhalteventil ausgerüstet war, wurden jeweils 2,5 kg Cyannorbornen mit einer Katalysatorkonzentration von 1,8 g Kobalt/kg in Form von Kobaltnaphthenat vorgelegt. Es wurde eine $H_2$/CO-Gasmischung der in nachstehender Tabelle angegebenen Zusammensetzung bis zu einem Druck von etwa 280 bar eingeleitet und die Reaktion unter weiterer Gaseinleitung bei etwa 160 bis 165°C durchgeführt. Die erhaltene Produktzusammensetzung ist aus der Tabelle ersichtlich.

Tabelle

Hydroformylierung von Cyannorbornenen bei verschiedener Synthesegaszusammensetzung

Katalysator: Kobalt-(iso)-octanoat

| Nr. | $H_2$ : CO | Zeit bis Beendigung der Gasaufnahme /Min./ | Produktionszusammensetzung /Gew.-%/ | | | |
|---|---|---|---|---|---|---|
| | | | CNB | FCNB | HCNB | Harz |
| 2 | 1 : 1 | 50 | 5,8 | 67,6 | 5,6 | 21,0 |
| 3 | 2 : 1 | 90 | 4,8 | 71,8 | 6,4 | 17,0 |
| 4 | 3 : 1 | 90 | 5,4 | 75,3 | 6,9 | 12,4 |

CNB = Cyannorbornen
FCNB = Formylcyannorbornan
HCNB = Hydroxymethylcyannorbornan

Unter den gleichen Reaktionsbedingungen wird - vergleichsweise - bei einem $H_2$/CO-Verhältnis von 1 : 3 folgende Produktzusammensetzung [Gew.-%] erhalten:

| CNB | FCNB | HCNB | Harz |
|---|---|---|---|
| 8,8 | 50,2 | 3,5 | 37,5 |

Beispiel 5

Hydroformylierung mit Rhodium

In einem 2 l-Autoklaven wurden 476 g (4,0 Mol) Cyannorbornen und 0,22 mMol Rhodiumoctanoat in 600 ml Toluol gelöst. Es wurden 250 bar einer Synthesegasmischung der Zusammensetzung $H_2$ : CO = 2,5 : 1 aufgedrückt; anschließend wurde der Reaktor erwärmt. Die Gasaufnahme begann bei 105°C, die Temperatur wurde auf ca. 120°C einreguliert. Sobald der Druck unter 200 bar sank, wurde mit Synthesegas der o. Zusammensetzung ergänzt. Die Gasaufnahme war nach 50 Minuten beendet. Das fast farblose Produkt wurde sofort über ein Steigrohr entnommen (585 g) und analysiert.
Die Zusammensetzung des Produktes war:

| | |
|---|---|
| Cyannorbornen | 2,5 % |
| Cyannorbornan | 0,7 % |
| Formylcyannorbornan | 87,2 % |
| Hydroxymethylcyannorbornan | 2,4 % |
| Harz | 7,2 % |

Die Ausbeute an Formylcyannorbornan war (nach Destillation) 509 g $\hat{=}$ 85,4 d. Th.

0065294
HOE 81/F 117

Patentansprüche:

1. Verfahren zur Herstellung von Formylcyannorbornan durch Hydroformylierung von Cyannorbornen mit einem $H_2$/CO-Gasgemisch in Gegenwart von fein verteilten Metallen der VIII. Gruppe des Periodensystems der Elemente, oder von Carbonylen oder carbonylbildenden Verbindungen dieser Metalle als Katalysatoren bei erhöhter Temperatur und erhöhtem Druck in Gegenwart oder in Abwesenheit eines inerten Lösungsmittels, dadurch gekennzeichnet, daß man als $H_2$/CO-Gasgemisch ein solches mit einem $H_2$/CO-(Mol)-Verhältnis von größer als 1, vorzugsweise von etwa 1,5 bis 5, insbesondere von etwa 1,5 bis 3, verwendet.

2. Verfahren nach Anspruch 1, dadurch gekennzeichnet, daß man als Katalysator fein verteiltes Rhodium, Rhodiumcarbonyl und/oder Rhodiumcarbonyl-bildende Verbindungen verwendet.

0065294

Europäisches Patentamt

# EUROPÄISCHER RECHERCHENBERICHT

Nummer der Anmeldung

EP 82 10 4243.9

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int Cl ) |
|---|---|---|---|
| D,A | US - A - 3 143 570 (EASTMAN KODAK)<br>* ganzes Dokument *<br>---- | 1,2 | C 07 C 121/46 |

RECHERCHIERTE SACHGEBIETE (Int Cl )

C 07 C 45/00
C 07 C 121/46

KATEGORIE DER GENANNTEN DOKUMENTE

X: von besonderer Bedeutung allein betrachtet
Y: von besonderer Bedeutung in Verbindung mit einer anderen Veroffentlichung derselben Kategorie
A: technologischer Hintergrund
O: nichtschriftliche Offenbarung
P: Zwischenliteratur
T: der Erfindung zugrunde liegende Theorien oder Grundsatze
E: alteres Patentdokument, das jedoch erst am oder nach dem Anmeldedatum veroffentlicht worden ist
D: in der Anmeldung angefuhrtes Dokument
L: aus andern Grunden angefuhrtes Dokument

&: Mitglied der gleichen Patentfamilie, ubereinstimmendes Dokument

X Der vorliegende Recherchenbericht wurde für alle Patentanspruche erstellt.

| Recherchenort | Abschlußdatum der Recherche | Prufer |
|---|---|---|
| Berlin | 20-07-1982 | BREW |